# EUROPEAN PATENT APPLICATION

(11) **EP 0 608 846 A2**
(43) Date of publication of application: **03.08.1994**
(21) Application number: 94101080.3
(22) Date of filing: 25.01.1994
(51) Int. Cl.: A61M 27/00

(54) **A medical drain**

(30) Priority: 28.01.1993 IL 10454593
(71) Applicant: Porat, Gadi, Jerusalem 93547 (IL)
(72) Inventor: Porat, Gadi, Jerusalem 93547 (IL)
(74) Representative: Benedum, Ulrich Max, Dr.

(57) **Abstract**

A medical drain tube for the removal of blood, secretions and other fluids or semifluids from surgical and inflammatory lesions and from other anatomical spaces wherein said drain is characterized by the ability to prevent or minimize an uncontrolled spillage and leakage of said fluids from the drain tube in the course of removing the drain from the patient's body at atmospheric pressure by means of unidirectional inlets along the tube's wall and a unidirectional valve or a plug at the distal opening of the drain tube, enabling the body fluids to flow in one direction from the outside into the inside of the tube.

## Description

### Field of the Invention

The present invention relates to a unidirectional surgical drain for the removal from the body of blood, secretions and other fluids or semifluids such as tissue fluids that originate in wounds, surgical or otherwise, in inflammatory lesions and exogenously administered fluids. The unidirectional drain of the present invention is specifically designed to prevent or minimize the backflow of its content under atmospheric pressure.

### Background of the Invention

Surgical drains are used mainly to prevent the accumulation of blood, plasma or other fluids within the body by allowing them to drain to the exterior. The drains consist of tubes usually made of silicone, PVC, latex, or other elastomer and plastics with inlets along the tube well. These inlets allow the passage of body fluids into the tube and out of the body. The drains exist in different configurations suitable for specific applications. The flow is driven by gravity or capillarity in systems such as the Penrose drains or by suction vacuum apparatus, either permanently fixed or portable. Removal of the drains form the body is very often associated with backflow of the drain content resulting in uncontrolled spillage of contaminated body fluids from the tube. It should be pointed out that this spillage is also associated with the suction driven drains where, to avoid pain to the patient, the vacuum is discontinued prior to the removal of the drain.

With the increasing awareness of transfection by means of contaminated body fluids, or semifluids or fluids administered to the body, particularly by the HIV and Hepatitis B Viruses, the absence of unidirectional drains, where the backflow and spillage of their contents is prevented, is rather surprising. The present invention offers a straightforward solution to this disadvantage.

### Summary of the Invention

The present invention relates to a medical drain tube for the removal of blood, secretions and other fluids or semifluids from the body following surgery, from inflammatory lesions and from other anatomical spaces. Said drain is characterized by the ability to prevent or minimize uncontrolled spillage and leakage of said body fluids from the drain tube in the course of removing the drain from the patient's body. This removal is done (after the vacuum is disconnected) at atmospheric pressure, and the leakage and spillage are prevented by means of unidirectional inlets along the tube wall and a unidirectional valve or plug at the distal opening of the drain tube, enabling the body fluids to flow only in the direction from the outside into the inside of the tube.

### Detailed Description of the Invention

The present invention relates to a medical drain tube for the removal from the body of blood, secretions and other fluids or semifluids following surgery, from inflammatory lesions and other anatomical spaces, wherein said drain is characterized by the ability to prevent an uncontrolled spillage and leakage of said fluids from the drain tube in the course of removing the drain from the patient's body at atmospheric pressure by means of unidirectional inlets along the tube's wall and a unidirectional valve or a plug at the distal opening of the drain tube, enabling the body fluids to flow only in one direction from the outside into the tube.

This invention relates only to the part of the drain tube which is located within the patient's body.

The above-mentioned terminology "unidirectional inlets" refers in the invention to any incisions or openings along the drain wall, or any conventional known or unknown medical one-way valve, enabling the flow of fluids during suction from the body into the drain tube, and preventing the spillage of those fluids out of the drain tube at atmospheric pressure, following disconnection of the suction device, when removing the drain tube from the patient's body. Said "unidirectional inlets" can be incisions passing through the drain tube's side wall such as cross-shaped incisions or tri-arm-shaped incisions. Said "unidirectional inlets" can be diagonal incisions that create a leaf valve. Said "unidirectional inlets" can be also an "overlap valve" along the drain tube side-wall, which consists of two partially overlapping layers of the tube wall - will hereinafter be referred to as an "overlap valve". In addition, "unidirectional inlets" may consist of a collapsible inner tube with or without a cross-shaped or tri-arm shaped incision or small holes through its wall, and said inner tube is inserted in a non-collapsible outer tube with regular side holes positioned in a manner securing that they do not coincide with the incisions or small holes in the inner tube wall - will hereinafter be referred to as "collapsible-tube valve".

The shape or number of inlets along the drain tube side-wall depend on the volume of fluid to be drained. Said inlets can be along one side or two sides or multiple sides of the tube. The preferred materials constructing said drain are silicon, PVC or latex. The distal end of the tube is either blocked or also has a unidirectional valve.

The present invention will be further clarified and exemplified by figures 1-10. This clarification illustrates only the preferred embodiment of the invention and in no way is intended to limit the scope of the invention.

### Brief Description of the Drawings

Figures 1a and 1b illustrate an isometric view of a round drain tube.
Figure 2a illustrates an isometric view of a drain tube with an overlap valve.
Figure 2b is a cross section profile view of said overlap drain tube.
Figure 3 illustrates a cross sectional profile view of the drain tube, with two overlap valves.
Figure 4a illustrates an isometric view of a drain tube with an unidirectional leaf valve.
Figure 4b illustrates a longitudinal section of said leaf valves in its closed position.
Figure 4c illustrates a longitudinal section of said leaf valves in its open position.
Figures 5a-c illustrate an isometric view of the separate parts of a drain tube that comprises a "collapsible-tube valve".

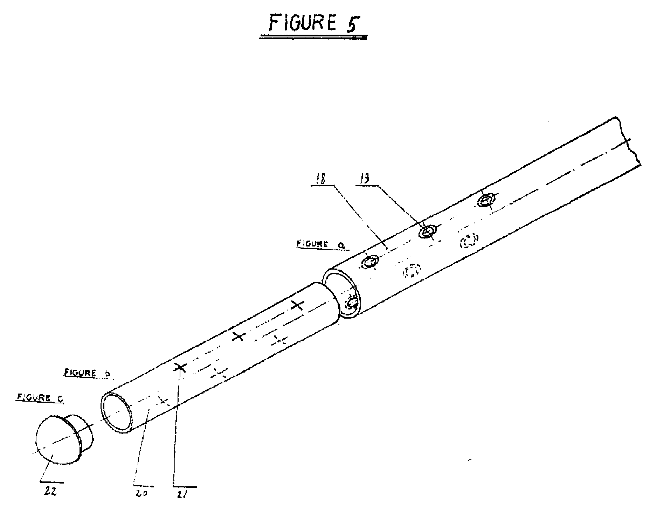

Figure 5a illustrates an isometric view of a longitudinal section of the non-collapsible outer tube.
Figure 5b illustrates an isometric view of the collapsible inner tube with cross-shaped incisions.
Figure 5c illustrates an isometric view of the plug (for the distal opening).

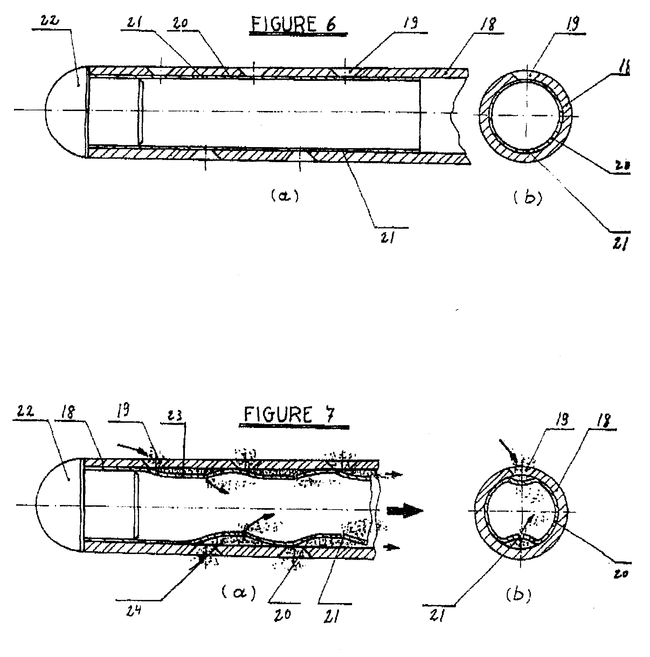

Figure 6a illustrates a longitudinal section through a drain tube that comprises a collapsible-tube valve with cross shaped incisions in its inactive closed position.
Figure 6b illustrates a cross-section of the drain described in figure 6a.
Figure 7a illustrates a longitudinal section through said collapsible-tube valve with the cross shaped incisions in its activated open position.
Figure 7b illustrates a cross-section of the drain described in figure 7a.

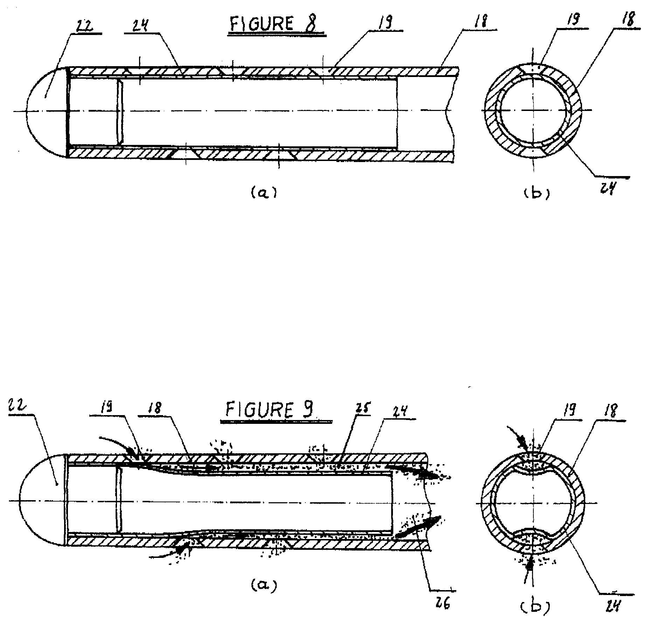

Figure 8a illustrates a longitudinal section through a drain tube that comprises a collapsible-tube valve (without incisions) in its inactive closed position.
Figure 8b illustrates a cross-section of the drain described in figure 8a.

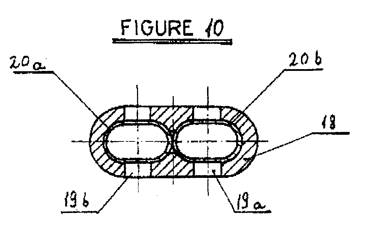

Figure 9a illustrates a longitudinal section through a drain tube that comprises a collapsible-tube valve (without incisions) in its activated open position.
Figure 9b illustrates a cross-section of the drain described in figure 9a.
Figure 10 illustrates a cross-section of a drain tube that comprises two collapsible-tube valves in its inactive closed position.

### Detailed Description of the Drawings

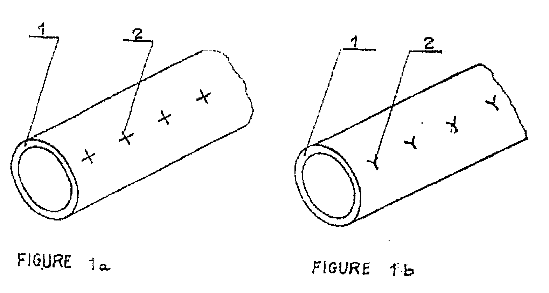

Figure 1 illustrates an example of a drain (1) with unidirectional inlets arranged as a plurality of incisions passing through the drain tube's side wall (2).

The number of cross-shaped incisions (or any other shape of incisions) will be determined according to the specific type of drain.

The cross-shaped incisions can be located along more than one side of the drain tube. The drain tube may have a round, oval, or "flat" profile or any other profile or cross-section. Longitudinal protrusions (anti-collapsing) may comprise an integral part of the tube wall.
Figure 1a demonstrates cross-shaped incisions.
Figure 1b demonstrates tri-arm shaped incisions.

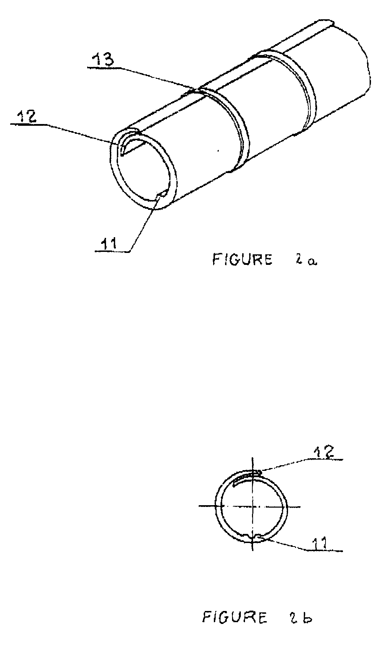

Figure 2a illustrates an isometric view of an "overlap valve" drain tube according to the present invention. An overlap valve (12) passes along the drain tube-wall. Said overlap valve (like the incisions) serves as a unidirectional inlet enabling the body fluids to flow only in one direction from the outside into the drain tube during suction, and in the course of removing the drain from the patient's body (at atmospheric pressure) said overlap valve tube prevents uncontrolled spillage or leakage. Said overlap valve version may have longitudinal protrusions (11) and rings (13) for reinforcing its structure. The tube can also be reinforced and/or strengthened by, for example, spiral strings, bands, a perforated jacket, etc.

Figure 2b illustrates a cross sectional profile view of said overlap drain tube with its overlap valve (12) and longitudinal protrusions (anti-collapsing) (11).

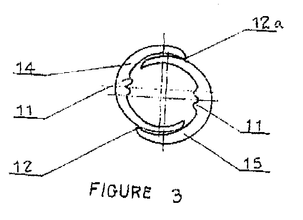

Figure 3 illustrates a cross sectional profile view of a "double overlap valve drain tube". This tube is constructed by a combination of two identical parts (14) and (15) which link up with one another, providing together two overlap valves (12) and (12a) along a drain tube. Said overlap version may have longitudinal protrusions (anti-collapsing) (11) and can be reinforced and/or strengthened by, for example, rings, spiral strings, bands, perforated jackets, etc.

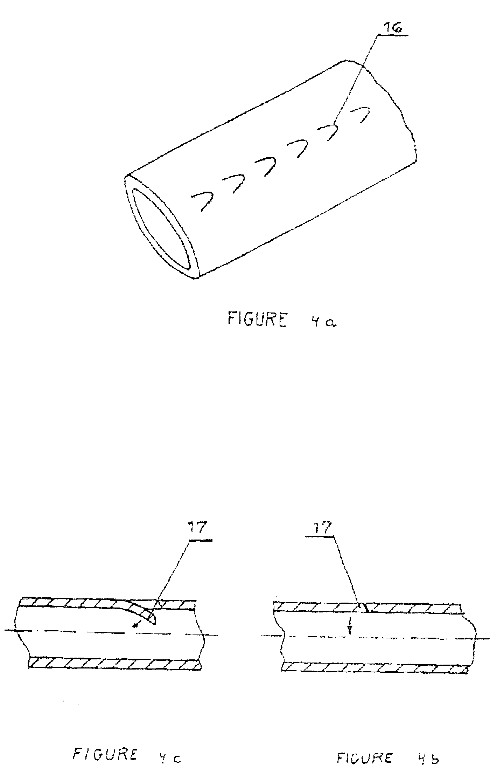

Figure 4a illustrates an isometric view of a drain with unidirectional leaf valve (16). Said loaf valves are diagonal incisions in the shape of a "leaf" allowing said "leaf" to bend only towards the inside of the tube (during suction).

Figure 4b illustrates a cross-section of said leaf valves (17) in its closed position.

Figure 4c illustrates a cross-section of said leaf valves (17) in its open position.

Figures 5a-c illustrate an isomeric view of the separate parts of a drain tube that comprises a "collapsible-tube valve".

Figure 5a illustrates an isometric view of a longitudinal section of the non-collapsible outer tube (18) with regular side holes (19).

Figure 5b illustrates an isometric view of the collapsible inner tube (20) with inlets arranged as a plurality of incisions (21) passing through the wall of said collapsible tube.

Figure 5c illustrates an isometric view of the plug (22) for the distal opening of the drain. This plug may be replaced by any unidirectional valve.

Figure 6a illustrates a longitudinal section through a collapsible-tube valve drain tube in its inactive closed position that comprises a collapsible inner tube (20) with trans-wall or small holes incisions (21). The non-collapsible outer tube (18) had regular side holes (19). The outer diameter of said inner collapsible tube conforms to the inner diameter of the outer non-collapsible tube. Said incisions or small holes through the wall of the inner collapsible tube do not coincide with the side holes of the outer non-collapsible tube. The plug or uni-directional valve (22) is plugged and sealed into the distal opening of the drain.

Figure 6b illustrates a cross-section of the collapsible-tube valve described in figure 6a. The inner collapsible tube (20) is located inside the outer non-collapsible tube (18). In the inactive closed position the regular side holes (19) of the outer tube are blocked by the inner tube and the openings in the inner tube (21) are blocked by the outer tube (18).

Figure 7a illustrates a longitudinal section through said collapsible-tube valve drain tube (with the small openings) in its activated open position. During suction, the inner collapsible tube (20) collapses (23) and the body fluids flow through the regular side holes (19) of the non-collapsible outer tube (18) and enters the space between the inner tube and the outer tube (24). Then, as a result of the suction and the low pressure, the body fluids can flow only in one direction through the incisions (21) from the outside of the inner tube into the inner tube and in the space between the inner tube and the outer tube.

Figure 7b illustrates a cross-section of the drain described in figure 7a.

Figures 8 and 9 illustrate a modification of the "collapsible-tube valve". The inner tube in this modification is without any openings, thus the body fluids flow only in the space created between the inner tube and the outer tube during suction.

Figure 8a illustrates a longitudinal section through a drain tube that comprises a collapsible-tube valve without incisions or any other unidirectional valves (24), an outer tube (18) with regular side holes (19) and a plug (22) for the distal opening of the drain. This plug may be replaced by any unidirectional valve.

Figure 8b illustrates a cross section of the drain described in figure 8a.

Figure 9a illustrates a longitudinal section trough said collapsible-tube valve (without incisions or any other openings) in its activated open position. During suction the inner tube (24) collapses and thus the body fluids flow from the regular side holes (19) of the non-collapsible outer tube (18) to the space created between the inner tube and the outer tube (25) during suction and then inside the drain (26).

Figure 9b illustrates a cross section of the drain described in figure 9a.

Figure 10 illustrates another embodiment of said invention. There may be more than one collapsible-tube valves. Figure 10 represents and example of two collapsible-tube valves (20a) and (20b) inside a non-collapsible outer tube (18) with regular side holes (19a) and (19b).

## Claims

1. A medical drain tube for the removal of blood, secretions and other fluids or semifluids from surgical and inflammatory lesions and from other anatomical spaces wherein said drain is characterized by the ability to prevent or minimize an uncontrolled spillage and leakage of said fluids from the drain tube in the course of removing the drain from the patient's body at atmospheric pressure by means of unidirectional inlets along the tube's wall and a unidirectional valve or a plug at the distal opening of the drain tube, enabling the body fluids to flow only in one direction from the outside into the inside of the tube.

2. A medical drain according to claim 1 wherein the unidirectional inlets consist of a plurality of incisions passing through the drain tube's wall.

3. A medical drain according to claim 2 wherein said incisions are cross shaped with the "cross" having three or more "arms".

4. A medical drain according to claim 1 wherein the unidirectional inlets are overlap valves wherein said valves consist of two partially overlapping layers of the tube wall.

5. A medical drain according to claim 4 wherein two or more overlap valves pass through and along the drain tube's wall and are constructed by a combination of two or more portions of the tube wall which are only partially closed and which link up with one another providing together said two or more overlap valves along the tube which they create.

6. A medical drain according to claim 4 or 5 wherein the tube is reinforced and/or strengthened by rings, spiral strings, bands or a perforated jacket with openings, or any other means for adhesion.

7. A medical drain according to claims 1 and 2 wherein the unidirectional inlets are leaf valves and wherein said leaf valves are diagonal incisions in the shape of a leaf allowing said "leaf" to bend only towards the inside of the tube.

8. A medical drain according to claim 1 wherein the medical drain tube has anti-collapsing longitudinal protrusions on the inner side of the tube wall.

9. A medical drain according to claim 1 wherein the unidirectional inlets are any conventional known medical one way valves.

10. A medical drain according to claims 1-3 wherein the unidirectional inlets consist of a plurality of incisions passing through the wall of an inner collapsible tube inserted in an outer non-collapsible tube with a plurality of side holes and wherein said incisions through the wall of the inner collapsible tube do not coincide with the holes in the wall of the outer non-collapsible tube and wherein the outer diameter of said inner collapsible tube conforms to the inner diameter of the outer non-collapsible tube.

11. A medical drain according to claim 10 wherein the inlets in the inner collapsible tube are regular holes.

12. A medical drain according to claims 1-3 wherein the unidirectional inlet consists of an inner collapsible tube (without incisions or regular holes) inserted in an outer non-collapsible tube with a plurality of side holes and wherein the outer diameter of said inner collapsible tube conforms to the inner diameter of the outer non-collapsible tube.

13. A medical drain according to claims 10-12 wherein there are two or more inner collapsible tubes.

14. A medical drain according to claim 1 made from silicon, latex or PVC, or any other Elastomer material or a combination of two or more of these materials.
